# EUROPÄISCHE PATENTSCHRIFT

(11) **EP 3 467 432 B1**
(45) Veröffentlichungstag und Bekanntmachung des Hinweises auf die Patenterteilung: **22.04.2020**
(21) Anmeldenummer: 18199891.5
(22) Anmeldetag: 04.02.2013
(51) Int. Cl.: G01B 11/25, A61C 9/00, A61B 5/107, G01B 21/04

(54) **VERFAHREN ZUM BETREIBEN EINER VORRICHTUNG ZUM ERFASSEN DER DREIDIMENSIONALEN GEOMETRIE VON OBJEKTEN**
METHOD FOR OPERATING A DEVICE FOR DETECTING THE THREE-DIMENSIONAL GEOMETRY OF OBJECTS
PROCÉDÉ DE FONCTIONNEMENT D'UN DISPOSITIF DESTINÉ À ENREGISTRER LA GÉOMÉTRIE TRIDIMENSIONNELLE D'OBJETS

(30) Priorität: 06.02.2012 DE 102012100953
(43) Veröffentlichungstag der Anmeldung: 10.04.2019
(62) Teilanmeldung aus: 13708646.8
(73) Patentinhaber: a.tron3d GmbH, 9020 Klagenfurt am Wörthersee (AT)
(72) Erfinder: JESENKO, Juergen, 9587 Riegersdorf (AT); NOWAK, Christoph, 1100 Wien (AT); KOINIG, Horst, 9020 Klagenfurt (AT)
(74) Vertreter: Beer & Partner Patentanwälte KG

(56) Entgegenhaltungen:
- US-B2- 7 068 825

## Beschreibung

Die Erfindung betrifft ein Verfahren zum Betreiben einer Vorrichtung zum Erfassen der dreidimensionalen Geometrie von Objekten, insbesondere Zähnen, mit einem Scanner mit einem Handstück mit wenigstens einer optischen Einrichtung mit wenigstens einer Kamera zum Aufnehmen von Bildern und mit wenigstens einer Lichtquelle, wobei vor einem Erfassen der dreidimensionalen Geometrie von Objekten mit dem Scanner aus verschiedenen bekannten Abständen Kalibrieraufnahmen einer vorzugsweise planen Fläche aufgenommen werden.

Ein derartiges Verfahren ist aus US 7,068,825 B2 bekannt.

Eine Vorrichtung zum Erfassen der dreidimensionalen Geometrie von Objekten ist beispielsweise aus der AT 508 563 B bekannt. Der Anwendungsbereich der Erfindung erstreckt sich dabei auf die Aufnahme von digitalen Zahn- und Kieferabdrücken, die Hilfestellung bei der Diagnose, die Überwachung von Zahnbehandlungen sowie die zuverlässige Kontrolle von eingesetzten Implantaten. Neben weiteren Einsatzgebieten im Bereich der Medizin- und Industrietechnik, beispielsweise im Bereich der Endoskopie, können auch Objekte stereometrisch vermessen werden, die schwer zugänglich sind.

Die Verwendung eines Lagesensors ist beispielsweise aus der US 5,661,519 A bekannt.

Solche Vorrichtungen können derart zu verbessert werden, dass sie mit möglichst geringer Stromversorgung zu betreiben sind. Angestrebt wird dabei ein Wert von zum Beispiel 500mA bzw. 900mA.

Aufgabe der Erfindung ist es, bei einer starren Montage aller Elemente der optischen Einrichtung, bei der es nicht möglich ist, die Optik der Kamera zu fokussieren, ausreichend gute Aufnahmen machen zu können.

Diese Aufgabe wird bei einem Verfahren der eingangs genannten Art dadurch gelöst, dass bei den Kalibrieraufnahmen ermittelte, von den Abständen abhängige Helligkeitsverläufe gemeinsam mit empirischen Werten von Rändern des Musters in einer Tabelle hinterlegt werden.

Durch die Anordnung des Mittels zum Erzeugen des Lichts direkt im Handstück werden lange optische Wege, beispielweise über Glasfaserkabel oder viele Umlenkspiegel, vermieden. Es wird dabei zwischen der Lichtquelle, also allem was Licht aussenden kann, beispielsweise das Ende eines Glasfaserkabels, und dem Mittel zum Erzeugen des Lichts, beispielsweise einem Laser oder dem Halbleiter einer LED, unterschieden.

Durch den Verzicht auf lange optische Wege kann ein Mittel zum Erzeugen des Lichts mit einer geringeren Leistung verwendet werden um das Objekt ausreichend auszuleuchten, was eine bemerkenswerte Energieersparnis bedeutet.

Die starre Montage aller Elemente der optischen Einrichtung bedeutet, dass es nicht möglich ist, die Optik der Kamera zu fokussieren. Alle Kalibrierungen der optischen Einrichtung erfolgen also im Vorfeld. Dabei ist es insbesondere wichtig eine optimale Einstellung der Blende zu erreichen. Eine kleinere Blende ist dabei gut für eine größere Schärfentiefe, bei größerer Blende wird eine geringere Ausleuchtung für eine ausreichend gute Aufnahme benötigt.

Mit unscharfen Bereichen in den 2D-Aufnahmen wird dabei auf zweierlei Art verfahren. Zum einen liefern Bereiche, in denen die 2D-Aufnahmen unscharf werden, Informationen über die Entfernung. So können anhand von bereits ermittelten Informationen über die Oberflächenkrümmung aus dem Grad der Unschärfe Tiefeninformationen gewonnen werden. Die unscharfen Bereiche können also als weitere Informationsquelle verwertet werden. Zum anderen können unscharfe Punkte, Flächen, Linien oder dergleichen scharf gezeichnet werden und so in den regulären, beispielsweise stereometrischen, Prozess der Gewinnung von dreidimensionalen Daten einfließen.

Hierfür wird der Scanner im Zuge des Kalibrierens beispielsweise über einer planen Ebene in verschiedenen bekannten Abständen angeordnet. Abstände die sich in Schritten von jeweils 50µm ändern, haben sich hierfür als besonders geeignet erwiesen. Es können aber auch andere Abstände zum Kalibrieren verwendet werden. Generell kann sich der Fachmann bei der Wahl der Abstände bzw. deren Änderungen an der Auflösung der verwendeten Mittel zum Erfassen der zweidimensionalen Bilder orientieren. Je besser Änderungen im erfassten zweidimensionalen Bild erkannt werden können, umso geringere Änderungen der Abstände zwischen Scanner und Ebene sind im Zuge des Kalibrierens sinnvoll.

Vor dem eigentlichen Erfassen der dreidimensionalen Geometrie von Objekten folgt daher erfindungsgemäß der Verfahrensschritt, dass aus verschiedenen bekannten Abständen mit dem Scanner Kalibrieraufnahmen einer vorzugsweise planen Fläche gemacht werden. Die Abstände ändern sich dabei in Schritten von bevorzugt 50µm. Weiters sind die Mittelachsen der Feldwinkel der Kameras während der Kalibrieraufnahmen bevorzugt im Wesentlichen normal zu der planen Fläche ausgerichtet.

Für jeden Abstand wird dabei jeweils ein mittlerer Helligkeitsverlauf von hellsten zu dunkelsten Bereichen der Punkte, Flächen, Linien oder dergleichen gespeichert. Bei einem späteren Aufbereiten von unscharfen zweidimensionalen Aufnahmen muss daher nicht mehr von einem statistischen Helligkeitsverlauf ausgegangen werden, um die Aufnahmen scharf zu zeichnen, sondern man kann wahrscheinliche Ränder aus einer beim Kalibrieren erstellten empirischen Tabelle ablesen. Die beim Scharfzeichen gewählten Ränder haben daher eine sehr viel größere Genauigkeit als aufgrund von herkömmlichen Verfahren gewählte Ränder. Weiters kann, da für einen Helligkeitsverlauf in einer zweidimensionalen Aufnahme ein möglichst ähnlicher Helligkeitsverlauf in der Tabelle gewählt wird, bereits vor der eigentlichen Auswertung der zweidimensionalen Aufnahmen abgeschätzt werden, wie weit der betreffende Bereich von der Kamera entfernt war, da für verschiedene Helligkeitsverläufe beim Kalibrieren verschiedene Abstände in der Tabelle hinterlegt wurden.

Kalibrieraufnahmen bei denen die Mittelachsen der Feldwinkel der Kameras um bekannte Winkel zur Fläche geneigt sind, sind ebenso denkbar.

In einer besonders bevorzugten Ausführungsform weist die Vorrichtung eine Einrichtung zum Synchronisieren der Energieversorgung von Lichtquelle und Kamera auf. So werden Kamera und Lichtquelle entsprechend einer bevorzugten Durchführungsform des Verfahrens synchron betrieben. Durch das Pulsen von Licht können punktuell große Leistungen bei verhältnismäßig geringem Energieaufwand erreicht werden. Bei dieser Ausführungsform wird auch auf Seiten der Aufnahme die Energieversorgung unterbrochen. So werden unbeleuchtete Aufnahmen vermieden und weiter Energie eingespart.

In einer weiters bevorzugten Ausführungsform weist das Handstück wenigstens einen Lagesensor, insbesondere einen Beschleunigungssensor, einen Magnetfeldsensor und/oder einen Neigungssensor auf. Mit diesem wird verfahrensgemäß ermittelt, wie groß die Änderung der räumlichen Lage der Vorrichtung ist, und daraus bestimmt, wie viele Aufnahmen von der Kamera in einer definierten Zeiteinheit gemacht werden sollen. So kann vermieden werden, dass bei geringer Bewegung mehr Bilder von ein und derselben Stelle gemacht werden, als für eine optimale Erfassung der Geometrie notwendig ist.

In diesem Sinne kann in einer bevorzugten Durchführungsform die Bildrate der aufgenommenen Bilder verändert werden, bevorzugt liegt die Bildrate zwischen 1 und 30 Bildern pro Sekunde.
Zusätzlich oder alternativ kann gemäß einer bevorzugten Durchführungsform des Verfahrens die Bildrate auch abhängig davon angepasst werden, ob eine größere oder geringere Stromversorgung zur Verfügung steht. So können bei größerer Stromversorgung mehr Lichtpulse ausgesendet und aufgenommen werden als bei niedrigerer Stromversorgung.

In einer möglichen Ausführungsform kann zusätzlich bestimmt werden, wie viele Bilder von einem definierten Bereich erfasst wurden. Aus diesem Wert kann einem aufgenommenen Bereich des Objektes eine Qualität zugeordnet werden, die gegebenenfalls in der 3D-Darstellung der Geometrie des Objektes wiedergegeben werden kann, so dass der Nutzer darauf reagieren kann. Bereiche, von denen nur wenige Daten erfasst wurden, die also eine größere Gefahr von Abweichungen von der Geometrie des Objektes aufweisen, können beispielsweise rot dargestellt werden. Bereiche, in denen die Anzahl der Aufnahmen bereits bei einem für die gewünschte Qualität ausreichenden Wert liegt, können beispielsweise grün dargestellt werden. Weitere Farben für Zwischenstufen sind ebenso denkbar wie für Bereiche, in denen bereits ein optimaler Wert erreicht ist, also weitere Aufnahmen keine wesentliche Verbesserung der erfassten Daten mehr bringen. Man kann natürlich auch nur Bereiche, die eine mindere Qualität haben, einfärben.

Im Sinne einer Energieersparnis kann nach einem zusätzlichen oder alternativen Verfahrensschritt für einen definierten Bereich ermittelt werden, wie viele Aufnahmen von diesem Bereich bereits gemacht wurden und ab Erreichen einer definierten Anzahl von Aufnahmen keine weiteren Aufnahmen dieses Bereiches machen. Diese Maßnahme eignet sich außerdem dazu, die benötigten Verarbeitungsschritte in einer Recheneinheit, welche die aufgenommenen Daten verarbeitet, zu optimieren, bzw. benötigte Rechenleistung einzusparen.

In einer bevorzugten Ausführungsform weist die optische Einrichtung wenigstens einen Projektor zur Projektion von Mustern auf. Die Projektion von Mustern verbessert die Möglichkeiten zur Erfassung der dreidimensionalen Geometrie.

In einer weiters bevorzugten Ausführungsform überdecken der Feldwinkel der Kamera und der Feldwinkel des Projektors einander zu wenigstens 50%, bevorzugt zu wenigstens 80%, besonders bevorzugt zu wenigstens 90%. Der Feldwinkel ist der kegelförmige Bereich, in dem die Projektion bzw. die Aufnahme erfolgt. Durch eine möglichst große Überschneidung wird ein möglichst großer Anteil der aufgewendeten Energie genutzt.

In einer bevorzugten Ausführungsform weist die Vorrichtung einen gegebenenfalls wieder aufladbaren elektrischen Energiespeicher auf. Dieser kann mehrere Funktionen erfüllen.

Zum einen kann er in einer bevorzugten Ausführungsform als alleinige Energiequelle der Vorrichtung dienen. In diesem Fall ist es sinnvoll, wenn die Vorrichtung weiters einen Datenspeicher oder eine Möglichkeit der kabellosen Datenübertragung aufweist. So kann das Gerät vollkommen frei ohne Kabel bewegt werden. In einer Ausführungsform, bei der die Daten gespeichert werden, ist es zweckmäßig, die spätere Übertragung der Daten, beispielsweise über einen USB-Anschluss, mit einem Aufladen des Energiespeichers zu verbinden.

Alternativ kann der Energiespeicher eine Hilfsstromquelle der Vorrichtung sein. Diese kann bei Bedarf zugeschalten werden. Dazu wird nach einem bevorzugten Verfahren zunächst ermittelt, wie viel Strom der Vorrichtung zur Verfügung steht. Im Ausführungsbeispiel ist im Speziellen vorgesehen, dass ermittelt wird, ob dem Gerät 500mA oder 900mA zur Verfügung stehen, also ob das Gerät an einem USB 2.0 oder einem USB 3.0 Port angeschlossen ist. Möchte man das Gerät also in einem Modus betreiben, der 900mA Stromversorgung benötigt, hat aber nur eine Stromversorgung von 500mA zur Verfügung, wird der Energiespeicher verfahrensgemäß als zusätzliche Energiequelle hinzu gezogen. Ähnlich kann analog dazu auch beim Anschluss an einen Low Power USB-Port, der üblicherweise mit 100mA betrieben wird, eine Stromversorgung von beispielsweise 500mA oder 900mA realisiert werden.

Alternativ oder zusätzlich kann in einer weiteren bevorzugten Durchführungsform aus dem ermittelten Wert der zur Verfügung stehenden Stromversorgung bestimmt werden, ob das Gerät gegebenenfalls mit zwei oder drei oder mehr Kameras betrieben werden soll. So werden für unterschiedliche Leistungen der Stromversorgung unterschiedliche Betriebsmodi geschaffen. Bevorzugt werden dabei zum Beispiel zwei Kameras in einem Betriebsmodus für 500mA und drei oder mehr Kameras in einem Betriebsmodus für 900mA betrieben.

In einer besonders bevorzugten Durchführungsform des Verfahrens werden die von der Kamera erfassten Daten ohne weitere Verarbeitung bzw. Aufbereitung an eine Recheneinheit oder ein Speichermedium weitergeleitet. So wird der Energieaufwand, der sonst für einen Prozessor bzw. Chip, der diese Verarbeitung bzw. Aufbereitung üblicherweise durchführt, vollkommen vermieden. Die weitere Verarbeitung in der Recheneinheit kann wenigstens teilweise in der CPU erfolgen, allerdings hat es sich gezeigt, dass es insbesondere im Hinblick auf die Schnelligkeit der Datenverarbeitung sinnvoll ist, einen Teil der für die Erfassung bzw. Berechnung der dreidimensionalen Geometrie erhobenen Daten in der GPU zu verarbeiten. So ist es möglich die Daten, insbesondere mittels der Kameras aufgenommene zweidimensionale Bilder ohne nennenswerten Zeitverlust direkt in eine dreidimensionale Darstellung auf einem Display bzw. eine auf einem Speichermedium verfügbare Datei (beispielsweise ein 3D-File im STL-Format) umzuwandeln.

Die Vorrichtung kann nach einer bevorzugten Ausführungsform ein thermovoltaisches Element aufweisen. Mit diesem kann gemäß einer bevorzugten Durchführungsform des Verfahrens aus der Wärme, welche beim Betrieb entsteht, elektrische Energie gewonnen werden. Diese kann dann zum einen direkt zum Betreiben der Vorrichtung verwendet werden, zum anderen kann aber, insbesondere beim Auskühlen des Gerätes auch ein Energiespeicher mit der gewonnenen Energie gespeist werden.

Bevorzugte Aus- und Durchführungsformen der Erfindung sind Gegenstand der abhängigen Ansprüche.

Die Erfindung wird in der Folge unter Bezugnahme auf die Zeichnungen weiter erläutert.
- Fig. 1: zeigt eine schematisierte Darstellung einer Ausführungsform der Vorrichtung und
- Fig. 2: zeigt eine schematische Ansicht der Unterseite einer Ausführungsform der Vorrichtung.

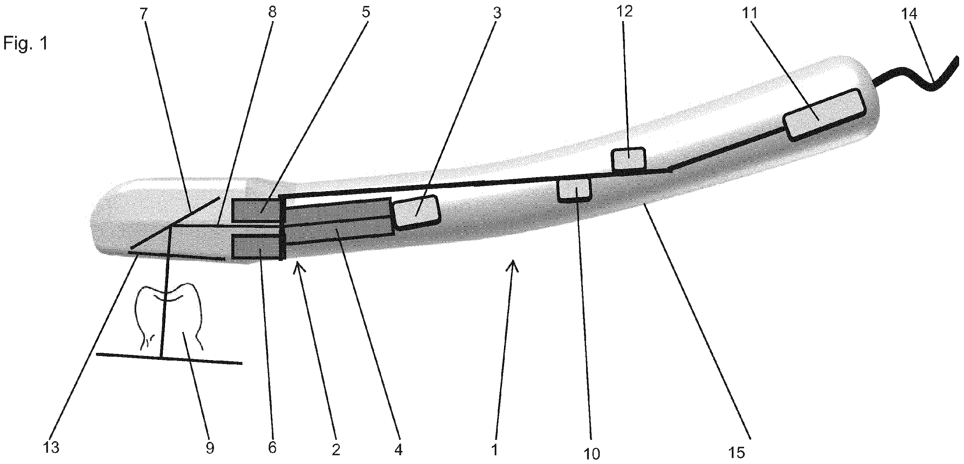

Die Fig. 1 zeigt eine beispielhafte Ausführungsform der Vorrichtung, bestehend aus einem Handstück 1, in welchem sich eine optische Einrichtung 2 befindet, welche eine Lichtquelle 3, einen Projektor 4, eine erste Kamera 5, eine zweite Kamera 6 sowie einen Spiegel 7 enthält. Vor dem Spiegel befindet sich im Gehäuse 15 des Handstücks 1 eine Ausnehmung. Diese ist aus hygienischen Gründen und zum Schutz der im Handstück 1 befindlichen Bauteile mit einer transparenten Abdeckung 13 versehen.

In dieser Ausführungsform ist die Lichtquelle 3 eine LED. Ein Mittel zum erzeugen des Lichts (in der Zeichnung nicht dargestellt) befindet sich in diesem Ausführungsbeispiel also in Form eines Halbleiters direkt in der Lichtquelle 3. Der weitere Verlauf des Lichts innerhalb und außerhalb der Vorrichtung ist durch einen beispielhaften Lichtstrahl 8 dargestellt.

Dieser durchläuft dabei zunächst den Projektor 4. Der Projektor 4 dient dabei der Projektion von Mustern auf das Objekt. Dabei kann es sich, abhängig von der Art der Erfassung der Geometrie, sowohl um regelmäßige Muster, wie beispielsweise Streifen, als auch um unregelmäßige Muster, wie beispielsweise unregelmäßige Punktemuster, handeln.
Nach dem Projektor 4 trifft der Lichtstrahl 8 auf den Spiegel 7 und wird über diesen auf das Objekt 9, dessen Geometrie erfasst werden soll, umgelenkt. Im dargestellten Ausführungsbeispiel handelt es sich bei dem Objekt 9 um einen Zahn. In einer in der Zeichnung nicht dargestellten Ausführungsform, bei der die Lichtquelle 3 und der Projektor 4 bereits in Richtung des Objektes ausgerichtet sind, kann auch auf den Spiegel 7 verzichtet werden.

Die Kameras 5,6 nehmen das auf den Zahn 9 projiziere Muster auf, aus welchem später die Geometrie des Zahns 9 errechnet wird. Gemäß einer bevorzugten Durchführungsform finden alle diesbezüglichen Berechnungen in einer Außerhalb des Handstücks 1 befindlichen Recheneinheit statt, wodurch der Stromverbrauch interner Chipsätze bzw. Prozessoren minimiert wird. Zu dieser Recheneinheit kann die Vorrichtung sowohl physisch mit einem Kabel 14 als auch drahtlos verbunden sein. Im Ausführungsbeispiel ist eine drahtlose Verbindung (beispielsweise Bluetooth oder WLAN) vorgesehen. Zu diesem Zweck befindet sich im Handstück ein Mittel zur drahtlosen Datenübertragung 10, insbesondere ein Sender und gegebenenfalls ein Empfänger.

Weiters ist im Handstück 1 ein gegebenenfalls wieder aufladbarer Energiespeicher 11 vorgesehen. Im dargestellten Ausführungsbeispiel dient sie als Hilfsstromquelle der Vorrichtung. Es kann aber auch vollständig auf ein Kabel am Handstück 1 verzichtet werden, wodurch optimale Bewegungsfreiheit gegeben ist.

Die Zeichnung zeigt außerdem einen Lagesensor 12. Mit diesem kann ermittelt werden, wie groß die räumliche Bewegung des Handstücks 1 ist. Zu diesem Zweck kann der Lagesensor 12 beispielsweise ein Beschleunigungssensor, ein Erdmagnetfeldsensor oder ein Neigungssensor sein. Kombinationen unterschiedlicher Sensortypen erhöhen dabei die Genauigkeit mit der die Änderung der räumlichen Lage bzw. die Bewegung des Handstücks 1 ermittelt wird.

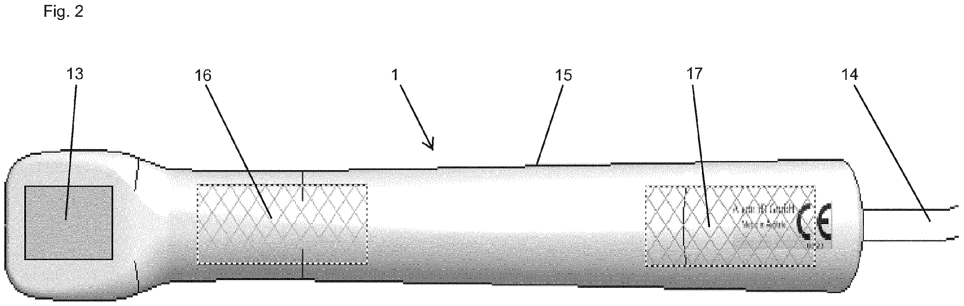

Die Fig. 2 zeigt eine schematische Ansicht der Unterseite auf eine Ausführungsform der Vorrichtung. Dabei sind zwei Bereiche 16, 17 gezeigt, in denen ein thermovoltaisches Element platziert werden könnte.

Im ersten Bereich 16, wird das thermovoltaische Element direkt an der Unterseite, also der Seite auf welcher sich die Abdeckung 13 befindet, in der Nähe der optischen Einrichtung 2 angeordnet. Dies ist vorteilhaft, da die optische Einrichtung 2, insbesondere der Projektor 4, während des Betriebs am meisten Wärme produziert und diese so mit möglichst geringen Verlusten genutzt werden kann.

Wird das thermovoltaische Element im zweiten Bereich 17 platziert, hat dies den Vorteil, dass es größer dimensioniert werden kann, allerdings wird dann ein Wärmeleiter, der die Wärme von der optischen Einrichtung 2 zum thermovoltaischen Element leitet, notwendig. Auch bei einer Positionierung des thermovoltaischen Elements im zweiten Bereich 17 ist eine Anbringung an der Unterseite des Handstücks 1 sinnvoll, damit eine gemäß einer bevorzugten Ausführungsform der Vorrichtung nach außen zeigende, Wärme abgebende Seite des thermovoltaischen Elementes nicht von der Hand des Nutzers abgedeckt wird.

## Patentansprüche

1. Verfahren zum Betreiben einer Vorrichtung zum Erfassen der dreidimensionalen Geometrie von Objekten, insbesondere Zähnen, mit einem Scanner mit einem Handstück (1) mit wenigstens einer optischen Einrichtung (2) mit wenigstens einer Kamera (5, 6) zum Aufnehmen von Bildern und mit wenigstens einer Lichtquelle (3), wobei vor dem Erfassen der dreidimensionalen Geometrie von Objekten mit dem Scanner aus verschiedenen bekannten Abständen Kalibrieraufnahmen einer vorzugsweise planen Fläche aufgenommen werden, **dadurch gekennzeichnet, dass** bei den Kalibrieraufnahmen ermittelte, von den Abständen abhängige Helligkeitsverläufe gemeinsam mit empirischen Werten von Rändern des Musters in einer Tabelle hinterlegt werden.

2. Verfahren nach Anspruch 1, **dadurch gekennzeichnet, dass** die Mittelachsen der Feldwinkel der Kameras während der Kalibrieraufnahmen im Wesentlichen normal zu der planen Fläche ausgerichtet sind.

3. Verfahren nach Anspruch 1, **dadurch gekennzeichnet, dass** die Mittelachsen der Feldwinkel der Kameras während der Kalibrieraufnahmen um bekannte Winkel zur Fläche geneigt sind.

4. Verfahren nach einem der Ansprüche 1 bis 3, **dadurch gekennzeichnet, dass** die Tabelle bei einem Scharfzeichnen zweidimensionaler Aufnahmen der Kamera (5, 6) im Zuge des Erfassens der dreidimensionalen Geometrie verwendet wird.

5. Verfahren nach einem der Ansprüche 1 bis 4, **dadurch gekennzeichnet, dass** mit einem Projektor (4) ein Muster auf das Objekt projiziert wird.

## Claims

1. Method for operating an apparatus for measuring the three-dimensional geometry of objects, in particular teeth, comprising a scanner which has a handpiece (1) having at least one optical device (2) which has at least one camera (5, 6) for capturing images, and comprising at least one light source (3), with calibration images of a preferably planar surface being captured from various known distances before the three-dimensional geometry of objects is measured by means of the scanner, **characterized in that** brightness curves which are determined during the calibration imaging process and which depend on the distances are recorded in a table together with empirical values of edges of the pattern.

2. Method according to claim 1, **characterized in that** the central axes of the field angles of the cameras are aligned so as to be substantially normal to the planar surface during the calibration imaging process.

3. Method according to claim 1, **characterized in that** the central axes of the field angles of the cameras are inclined by known angles with respect to the surface during the calibration imaging process.

4. Method according to any of claims 1 to 3, **characterized in that** the table is used when sharpening two-dimensional images from the camera (5, 6) in the course of measuring the three-dimensional geometry.

5. Method according to any of claims 1 to 4, **characterized in that** a pattern is projected onto the object by means of a projector (4) .

## Revendications

1. Procédé de fonctionnement d'un dispositif destiné à enregistrer la géométrie tridimensionnelle d'objets, en particulier de dents, au moyen d'un scanner doté d'une pièce à main (1) avec au moins un moyen optique (2) avec au moins une caméra (5, 6) permettant de prendre des photos et avec au moins une source de lumière (3), selon lequel des enregistrements d'étalonnage d'une surface de préférence plane sont effectués à partir de différentes distances connues avant l'enregistrement de la géométrie tridimensionnelle des objets au moyen du scanner, **caractérisé en ce que** des courbes de brillance déterminées en fonction des distances sont consignées, conjointement avec des valeurs empiriques de bords du motif, dans un tableau pendant les enregistrements d'étalonnage.

2. Procédé selon la revendication 1, **caractérisé en ce que** les axes centraux des angles de champ des caméras sont alignés essentiellement de manière normale par rapport à la surface plane lors des enregistrements d'étalonnage.

3. Procédé selon la revendication 1, **caractérisé en ce que** les axes centraux des angles de champ des caméras sont inclinés suivant des angles connus par rapport à la surface lors des enregistrements d'étalonnage.

4. Procédé selon l'une des revendications 1 à 3, **caractérisé en ce que** le tableau est utilisé pour augmenter la netteté d'enregistrements bidimensionnels de la caméra (5, 6) au cours de l'enregistrement de la géométrie tridimensionnelle.

5. Procédé selon l'une des revendications 1 à 4, **caractérisé en ce qu'**un motif est projeté sur l'objet au moyen d'un projecteur (4).
